# EUROPÄISCHE PATENTSCHRIFT

(11) **EP 1 572 175 B1**
(45) Veröffentlichungstag und Bekanntmachung des Hinweises auf die Patenterteilung: **15.11.2006**
(21) Anmeldenummer: 03779907.9
(22) Anmeldetag: 13.11.2003
(51) Int. Cl.: A61K 31/04, A61P 41/00, A61K 36/82, A61K 31/198

(54) **GASTRO-INTESTINAL VERABREICHBARE FORMULIERUNG AUS GRÜNTEE-EXTRACKT UND EINEN NO-DONOR**
FORMULATION, WHICH CAN BE ADMINISTERED GASTROINTESTINALLY, CONTAINING GREEN TEA EXTRACT AND AN NO DONOR
FORMULATION ADMINISTRABLE PAR VOIE GASTRO-INTESTINALE, CONTENANT UN EXTRAIT DE THE VERT ET UN DONNEUR D'OXYDE NITRIQUE (NO)

(30) Priorität: 09.12.2002 DE 10257360
(43) Veröffentlichungstag der Anmeldung: 14.09.2005
(73) Patentinhaber: Fresenius Kabi Deutschland GmbH, 61352 Bad Homburg v.d.H. (DE)
(72) Erfinder: SCHNEIDER, Heinz, CH-1792 Cordast (CH)
(74) Vertreter: Ackermann, Joachim
(86) Internationale Anmeldenummer: PCT/EP2003/012675
(87) Internationale Veröffentlichungsnummer: WO 2004/052352

(56) Entgegenhaltungen:
- EP-A- 0 875 155
- WO-A-91/18610
- US-A- 5 902 829

## Beschreibung

### Gastro-intestinal verabreichbare Formulierung und deren Verwendung

Die vorliegende Erfindung betrifft die Verwendung einer gastro-intestinal verabreichbaren Arznei- oder Nährstoff-Formulierung sowie deren Verwendung zur Vermeidung oder Verringerung des Risikos postoperativer Komplikationen.

Bei schweren operativen Eingriffen ist häufig mit postoperativen Komplikationen zu rechnen. Diese können verschiedene Symptome aufweisen, lassen sich aber häufig auf den Umstand zurückführen, dass das von der Operation betroffene Gewebe nicht ausreichend durchblutet wird. Sogenannte Ischemie / Reperfusions-Phänomene können bei Operationspatienten zu lebensbedrohenden Zuständen führen.

Bislang wurden derartige Komplikationen hauptsächlich erst dann behandelt, wenn diese bereits aufgetreten waren.

Es sind allerdings auch schon Ansätze beschreiben worden, bei denen ein Patient vor einer Operation durch Gabe von ausgewählten Formulierungen vorbereitet wird, um das Risiko des Auftretens von bzw. die Auswirkungen von postoperativen Komplikationen zu verringern.

So beschreibt die US-A-5,656,608 ein Verfahren zur Behandlung der Endotoxemie umfassend die Verabreichung einer wirksamen Menge einer ausgewählten Aminosäure, wie Glycin, Alanin oder Serin. Die Zugabe erfolgt mindestens drei Tage vor der Operation.

Aus der US-A-5,731,290 ist Verfahren zur Verbesserung der Immunantwort oder der Resistenz gegenüber Infektionen nach operativen Eingriffen bekannt, wobei ein Nahrungsmittelzusatz präoperativ verabreicht wird. Dieser weist eine das Immunsystem stimulierende Menge von omega-3-Fettsäuren (hochungesättigten Fettsäuren) kombiniert mit L-Arginin, L-Ornithin oder deren Vorläufer auf. Die Verarbreichung des Zusatzes erfolgt über eine Zeitdauer von mindestens drei Tagen vor der Operation.

Die WO-A-96/25,861 beschreibt die Verwendung von Glycin oder der Glycinvorläufer Alanin und Serin zur Herstellung eines Medikaments bzw. einer Nahrungsmittelformulierung zur Verminderung des Gehaltes an Tumor-Nekrose-Faktor (TNF) bei Patienten, bei denen Homöostase bzw. lokale Entzündungen vorliegen. Diese Schrift erwähnt die Möglichkeit der Zugabe über eine Zeitdauer von mindestens drei Tagen vor einer Operation.

Aus der WO-A-99/62,508 ist die Verwendung von Glycin zur Herstellung eines Medikaments für die Behandlung von hämorrhagischem Schock bekannt. Es wird beschrieben, dass eine präoperative Verabreichung möglich ist.

Aus der US-A-5,902,829 ist ein Verfahren zur Beeinflussung der Mikrozirkulation bei Patienten bekannt, worin L-Arginin oder ein Vorläufer davon oder ein anderer NO-Donor, der ein Substrat der NO-Synthetase ist bzw. ein Vorläufer davon präoperativ verabreicht werden. Die Zugabe erfolgt über eine Zeitdauer von mindestens einem Tag vor der Operation.

In der US-A-6,013,273 wird ein Verfahren zur Behandlung von endotoxischem Schock beschrieben, welches die Zuführung einer wirksamen Menge von Cholin beinhaltet. Die Verabreichung erfolgt über eine Zeitdauer von mindestens einem Tag vor der Operation und in der Regel ein bis sechs Tage vor der Operation.

Allen diesen Behandlungen ist gemeinsam, dass sie mindestens einen Tag, in der Regel mehrere Tage vor einer Operation vorgenommen werden müssen. Bei Patienten mit akutem Operationsbedarf oder in Notfällen (z.B. Traumata) reicht die zur Verfügung stehende Zeit häufig nicht aus, um mit den bekannten Methoden ein zufrieden stellendes Ergebnis zu erzielen.

Ausgehend von diesem Stand der Technik wird mit der vorliegenden Erfindung eine Formulierung bereitgestellt, mit der ein Patient kurzfristig vor einer Operation unterstützt werden kann, um das Risiko des Auftretens von bzw. die Auswirkungen von postoperativen Komplikationen zu verringern.

Ferner wird mit der vorliegenden Erfindung ein Mittel zur Prophylaxe von postoperativen Komplikationen bereitgestellt, das insbesondere auf die Unterstützung von Notfallpatienten abzielt.

Die erfindungsgemäß bereitgestellte Formulierung das kann in solchen Fällen zum Einsatz gelangen, in denen nur noch ein geringer Zeitraum, beispielsweise einige Stunden, zur Verfügung bleibt; sie können natürlich auch in längeren Zeiträumen vor einer Operation zum Einsatz kommen.

Die erfindungsgemäß bereitgestellte Formulierung enthält als einen Bestandteil Grüntee-Extrakt. Bereits seit langem ist bekannt, dass Grüntee-Extrakt bei der Behandlung bestimmter Krankheiten eingesetzt werden kann und dass dieser Extrakt antibakterielle Eigenschaften aufweist.

So beschreibt die JP-A-73/30,599 den Einsatz von Polyphenolen aus Grüntee zur Krebstherapie.

Aus Neuroscience Letters 287 (2000), 191-4 ist die schützende Wirkung von (-)-Epigallocatechingallat, dem Hauptbestandteil der Grüntee-Polyphenole gegen Ischämie/Reperfusionsschäden im Hirn bekannt.

Aus Am. J. Physiol. Gastrointest. Liver Physiol., 2002, 283(4): G957-64, ist bekannt, dass Ischämie/Reperfusionsschäden der Leber durch Gabe von Grünteeextrakt vorgebeugt werden kann.

Die JP-A-101248,538 beschreibt eine Zusammensetzung zur Erhöhung der Aktivität der alpha-Wellen des Gehirns umfassend Grünteeextrakt, Aminosäuren, Vitamine und Zucker.

Aus der EP-A-1,174,143 ist der Einsatz von Grüntee-Extrakt zur Verwendung bei der Behandlung von durch Cyclosphorine oder Ascomycine induziertem Nierenversagen bekannt. Diese Schrift offenbart ferner Zusammensetzungen, die Grüntee-Extrakt und ausgewählte Aminosäuren, wie Glycin, L-Alanin, L-Serin oder L-Arginin enthalten.

In "Chemistry and Applications of Green Tea" (T. Yamamoto et al.) wird beschrieben, dass Grüntee-Extrakt eine günstige Wirkung auf die Darmflora besitzt und das Immunsystem positiv beeinflusst.

Ferner beschreiben F. Yang et al. in J. Nutr., 1998, 128: 2334-2340 "Green Tea polyphenols block endotoxin-induced TNF-production and lethality in a murine model", dass Polyphenole aus dem Grüntee entzündungshemmende und Antikrebs-Eigenschaften aufweisen und dass die Gabe von Grüntee-Polyphenolen den Gehalt an TNF-alpha im Serum reduziert.

Es wurde nun überraschenderweise gefunden, dass durch Verabreichung einer Zusammensetzung enthaltend Grüntee-Extrakt und ausgewählte weitere Komponenten, wie ausgewählte Aminosäuren oder Aminosäurederivate, die Unterstützung von Operationspatienten möglich ist, und dass damit das Risiko von postoperativen Komplikationen deutlich gesenkt und der Verlauf derartiger Komplikationen erheblich in ihren Auswirkungen reduziert werden kann.

Die vorliegende Erfindung betrifft die Verwendung einer Zusammensetzung enthaltend
a) Grüntee-Extrakt und
b) mindestens einen NO-Donor, der ein Substrat der NO-Synthetase ist, und/oder mindestens einen Vorläufer dieses NO-Donors,
zur Herstellung einer Formulierung zur gastro-intestinalen Verabreichung vor chirurgischen Eingriffen, um das Risiko für postoperative Komplikationen zu verringern oder solches zu vermeiden.

Unter dem Begriff gastro-intestinal verabreichbare Formulierung wird im Rahmen dieser Beschreibung eine Formulierung verstanden, die an beliebiger Stelle des Gastro-Intestinaltraktes verabreicht werden kann. Bevorzugte Darreichungsformen sind die orale Einnahme oder die Zuführung mittels Magensonde oder Darmsonde.

Unter einem chirurgischen Eingriff sind im Rahmen dieser Beschreibung beliebige chirurgische Eingriffe zu verstehen, vorzugsweise jedoch elektive chirurgische Eingriff oder chirurgische Notfalleingriffe.

Beispiele für elektive chirurgische Eingriffe sind gastrointestinale Eingriffe, Herzchirurgie, Hals- und Nasenchirurgie, Abdominaleingriffe, einschließlich minimal invasiver Abdominaleingriffe, Gefäß- und Gelenkchirurgie oder Transplantationen.

Beispiele für chirurgische Notfalleingriffe sind traumachirurgische Eingriffe oder Eingriffe zur Sanierung eines septischen Fokus.

Die erfindungsgemäß verwendete Formulierung wird vorzugsweise wenige Tage vor einem chirurgischen Eingriff, beispielsweise zwischen dem dritten präoperativen Tag und der Operation verabreicht. Bevorzugt erfolgt die Verabreichung innerhalb von vierundzwanzig Stunden vor der Operation, insbesondere weniger als zwölf Stunden, besonders bevorzugt weniger als sechs Stunden, und ganz besonders weniger als drei Stunden vor der Operation.

Die erfindungsgemäß verwendete Formulierung kann neben den Komponenten a) und b) als optionale Komponente c) Glycin, einen Glycin-Vorläufer, vorzugsweise in Form eines Di- oder Tripeptids, der physiologisch verträglichen Salze davon oder deren Kombinationen enthalten.

Neben den Komponenten a), b) und gegebenenfalls c) kann die erfindungsgemäße Formulierung noch weitere Komponenten enthalten, beispielsweise Geschmacksstoffe oder Aromen sowie Füllstoffe (z.B. Organoleptika) und Farbstoffe.

Die beschriebene Formulierung wird dem Patienten gastro-intestinal verabreicht, vorzugsweise oral oder mittels Magen- bzw. Darmsonde.

Oral verabreichbare Formulierungen können zum Beispiel in Form von Pillen, Tabletten, Filmtabletten, Brausetabletten (zur Herstellung von wässrigen Lösungen, Emulsionen oder Suspensionen), Dragees, Granulaten, Hart- und Weichgelatinekapseln, wässrigen, alkoholischen oder öligen Lösungen, Sirupen, Emulsionen oder Suspensionen zum Einsatz kommen.

Bei der Verabreichung mittels Magen- bzw. Darmsonde werden die Formulierungen in der Regel in Form von wässrigen, alkoholischen oder öligen Lösungen, Sirupen, Emulsionen oder Suspensionen verabreicht.

Die Herstellung der Formulierungen kann nach den bekannten Standardverfahren erfolgen.

Dazu werden die Komponenten a), b) und gegebenenfalls c) zusammen mit einem oder mehreren festen oder flüssigen galenischen Trägerstoffen und/oder Zusatzstoffen oder Hilfsstoffen sowie Energieträgern, wie Kohlenhydraten, Fetten und /oder Eiweißen und, wenn gewünscht, in Kombination mit anderen Nährstoffen mit prophylaktischem Nutzen in eine geeignete Verabreichungsform bzw. Dosierungsform gebracht, die dann in der erfindungsgemäßen Weise verwendet werden kann.

Die Formulierungen enthalten eine prophylaktisch nützliche Dosis der Komponenten a), b) und gegebenenfalls c), die normalerweise 0,5 bis 90 Gewichtsprozent der Formulierung ausmacht.

Für die Herstellung beispielsweise von Pillen, Tabletten, Dragees und Hartgelatinekapseln kann man Lactose, Stärke, zum Beispiel Maisstärke, oder Stärkederivate, Talk, Stearinsäure oder deren Salze verwenden. Trägerstoffe für Weichgelatinekapseln sind zum Beispiel Fette, Wachse, halbfeste und flüssige Polyole, natürliche oder gehärtete Öle. Als Trägerstoffe für die Herstellung von Lösungen oder von Emulsionen bzw. Suspensionen oder Sirupen eignen sich beispielsweise Wasser, physiologische Kochsalzlösung, Alkohole wie Ethanol, Glycerin, Polyole, Saccharose, Invertzucker, Glucose, Mannit, pflanzliche Öle.

Komponenten a), b) und gegebenenfalls c) können auch lyophilisiert werden und die erhaltenen Lyophilisate zum Beispiel zur Herstellung von Infusionspräparaten verwendet werden. Als Trägerstoffe für Mikrokapseln, Implantate oder Rods eignen sich zum Beispiel Mischpolymerisate aus Glykolsäure und Milchsäure.

Die Formulierungen können neben den Komponenten a), b) und gegebenenfalls c) noch Trägerstoffe sowie weitere übliche Zusatzstoffe enthalten, zum Beispiel Füllstoffe, Spreng-, Binde-, Gleit-, Netz-, Stabilisierungs-, Emulgier-, Dispergier-, Konservierungs-, Süß-, Färbe-, Geschmacks- oder Aromatisierungsmittel, Verdickungsmittel, Verdünnungsmittel, Puffersubstanzen, ferner Lösungsmittel oder Lösungsvermittler oder Mittel zur Erzielung eines Depoteffekts, Salze zur Veränderung des osmotischen Drucks, Überzugsmittel, Vitamine oder Antioxidantien.

Besonders bevorzugt kommen die Formulierungen in der Form von wässrigen Emulsionen, Suspensionen oder insbesondere Lösungen zum Einsatz.

Die Dosierung der zu verabreichenden Komponenten a), b) und gegebenenfalls c) hängt vom Einzelfall ab und ist wie üblich für einen optimalen Nutzen den individuellen Gegebenheiten anzupassen. So hängt sie ab von der Art und Ausmaß der zu erwartenden Komplikationen sowie von Geschlecht, Alter, Gewicht und individueller Ansprechbarkeit des zu behandelnden Menschen, sowie von der Bioverfügbarkeit der prophylaktisch verabreichten Komponenten.

Typische Gehalte an Komponente a) bewegen sich im Bereich von 0,01 bis 2,0 g, vorzugsweise im Bereich von 0,1 bis 1,5 g, jeweils bezogen auf 1000 ml einer Formulierung. Die Gewichtsangaben beziehen sich auf die im Grüntee-Extrakt enthaltenen Polyphenole.

Typische Gehalte an Komponente b) bewegen sich im Bereich von 1 bis 150 g, vorzugsweise im Bereich von 0,1 bis 30 g, jeweils bezogen auf 1000 ml der Formulierung.

Typische Gehalte an Komponente c) bewegen sich im Bereich von 0,01 bis 80 g, vorzugsweise im Bereich von 0,1 bis 30 g, jeweils bezogen auf 1000 ml der Formulierung.

Der Rest der Formulierung besteht vorzugsweise aus Wasser.

Die Formulierungen beinhalten üblicherweise einen Energiegehalt von weniger als 800 kcal, vorzugsweise 100 bis 500 kcal, jeweils bezogen auf 1000 ml.

Energiereiche Zusätze, wie Kohlenhydrate und/oder Fette und/oder Eiweiße sind vorzugsweise nicht vorhanden. Zur Verbesserung des Geschmacks können allerdings geringe Mengen, beispielsweise 1 bis 120 g/1000 ml, von Kohlenhydraten und/oder Fetten und/oder Eiweißen zugegen sein.

Typische Mengen der während der gesamten Behandlungsdauer zugeführten Komponente a) bewegen sich im Bereich von 5 bis 2000 mg, vorzugsweise 10 bis 1600 mg, bezogen auf den Polyphenolgehalt von Komponente a).

Typische Mengen der während der gesamten Behandlungsdauer zugeführten Komponente b) bewegen sich im Bereich von 0,1 bis 50 g, bezogen auf die einzelnen freien Aminosäuren.

Typische Mengen der während der gesamten Behandlungsdauer zugeführten Komponente c) bewegen sich im Bereich von 0,1 bis 40 g, bezogen auf die freie Aminosäure.

Die Formulierung kann in einer Einzeldosis verabreicht werden oder, insbesondere bei der Applikation größerer Mengen, in mehrere, zum Beispiel zwei, drei oder vier Einzeldosen aufgeteilt werden. Gegebenenfalls kann es, je nach individuellem Verhalten, erforderlich werden, von der angegebenen Dosis nach oben oder nach unten abzuweichen.

Als Grüntee-Extrakt können beliebige und handelsübliche Extrakte zum Einsatz kommen. Dabei kann es sich um wasser- oder um öllösliche Grüntee-Extrakte handeln.

Der erfindungsgemäß eingesetzte Grüntee-Extrakt enthält vorzugsweise die üblicherweise im Grüntee enthaltenden Bestandteile sind. Beispiele dafür sind Aminosäuren, Polyphenole, Vitamine, Saccharide, Mineralstoffe und Koffein.

Zu den bevorzugten im Grüntee-Extrakt enthaltenen Aminosäuren zählt Theanin.

Zu den bevorzugten im Grüntee-Extrakt enthaltenen Polyphenolen zählen die Derivate des Catechins, also (-)-Epigallocatechingallat (EGCg), (-)-Epigallicatechin (EGG), (-)-Epicatechingallat (ECg), (+)-Gallocatechin (GC), (-)-Epicatechin (EC) und (+)-Catechin (C) sowie Mischungen von zwei oder mehreren dieser Komponenten.

Als Grundlage für erfindungsgemäß einzusetzenden Grüntee-Extrakte können kommerziell erhältliche Produkte eingesetzt werden. Beispiele dafür sind die Produkte Sunphenon 100S, Sunphenon DCF-1, Sunphenon BG, Sunphenon LA-50, und Sunkatol (alle von Taiyo Kagaku Corp., Japan erhältlich).

Der erfindungsgemäß eingesetzte Grüntee-Extrakt kann durch Behandeln von Blättern des Grüntees mit heißem Wasser von beispielsweise 80 bis 95°C erhalten werden. Dabei werden in der Regel fünf bis zehn Gewichtsteile Wasser auf einen Gewichtsteil Teeblätter eingesetzt. Die Extraktionsdauer beträgt üblicherweise zehn bis 40 Minuten. Nach erfolgter Extraktion wird abfiltriert und das Filtrat wird im Vakuum konzentriert, beispielsweise auf ein Viertel des ursprünglichen Volumens, oder bis zum vollständigen Verdampfen des Wassers, so dass ein Pulver entsteht. Dieses Filtrat bzw. Pulver kann unmittelbar zur Herstellung der erfindungsgemäßen Formulierung eingesetzt werden.

Anstelle davon lässt sich auch ein Konzentrat einsetzen. Dieses kann durch mehrmalige Extraktion aus dem oben beschriebenen Filtrat mittels eines geeigneten Extraktionsmittels, beispielsweise Essigsäureethylester, gewonnen werden.

Dazu wird das oben beschriebene Filtrat bzw. das Konzentrat beispielsweise mehrmals mit einem etwa gleichen Volumen von Essigsäureethylester ausgeschüttelt, um die wirksamen Bestandteile des Grüntees zu extrahieren. Die vereinigten Extrakte werden im Vakuum bis zur Gewichtskonstanz konzentriert. Der erhaltene Rückstand kann als Komponente a) eingesetzt werden.

Zum Einsatz in der erfindungsgemäßen Formulierung kommt Grüntee-Extrakt in getrockneter oder flüssiger Form zum Einsatz, beispielsweise in Form eines Pulvers, eines Öls oder einer wässrigen Lösung. Vorzugsweise werden wässrige Lösungen eingesetzt.

Vorzugsweise weist der eingesetzte Grüntee-Extrakt Theanin und Polyphenole auf, die sich von Catechinderivaten, insbesondere den oben als bevorzugt beschriebenen Catechinderivaten, ableiten.

Als NO-Donoren, die ein Substrat der NO-Synthetase sind, und/oder als deren Vorläufer können unterschiedlichste Verbindungen eingesetzt werden.

Beispiele dafür sind Aminosäuren mit Aktivität als Substrat für die NO-Synthetase, insbesondere Arginin und Glutamin, Vorläufer dieser Aminosäuren, deren physiologisch verträgliche Salze oder Kombinationen dieser Verbindungen.

Ebenfalls bevorzugt als NO-Donoren, die Substrate der NO-Synthetase sind, werden eingesetzt Trinitroglycerin, Isosorbitdinitrat, Nitroprussit, Aminoguanidin, Spermin-NO, Spermidin-NO und SIN 1 (3-morpholinosydnonimine).

Unter dem Begriff Vorläufer von Aminosäuren werden Verbindungen verstanden, welche die betreffende Aminosäure oder deren Vorläufer enthalten, und welche durch Stoffwechselaktivitäten zur Freisetzung der betreffenden Aminosäure führen.

Beispiele für Aminosäure-Vodäufer sind Derivate der Aminosäure, wie Ester, Amide, N-alkylierte bzw. N-acylierte Aminosäure, Salze oder Keto-Vorläufer davon, sowie kurzkettige Peptide, wie Di- bis Decapeptide, vorzugsweise Tripetide, und ganz besonders bevorzugt Dipeptide, welche die betreffende Aminosäure enthalten. Beispiele für Tripeptide sind X-AS-X', X-X'-AS und X-AS-AS, wobei X und X' für natürlich vorkommende Aminosäuren stehen und AS für die betreffende Aminosäure steht.

Bevorzugt werden Derivate von Aminosäuren in der Form von Tri- und insbesondere Dipeptiden eingesetzt.

Beispiele für bevorzugte Derivate des Arginins sind die Dipeptide Ala-Arg, Arg-Ala, Arg-Gly und Gly-Arg.

Beispiele für bevorzugte Derivate des Glutamins sind die Dipeptide Ala-Gln und Gly-Gln.

Beispiele für bevorzugte Derivate des Glycins sind die Dipeptide Ala-Gly, Gly-Ala und Gly-Gly.

Beispiele für physiologisch verträgliche Salze sind Phosphate, Citrate, Acetate, Malate, Tartrate, Fumarate, Lactate und Hydrate.

Bevorzugt werden Zusammensetzungen eingesetzt, bei denen Komponente b) Arginin oder ein Arginin-Vorläufer in Form eines Di- oder Tripeptids ist.

Das nachstehende Beispiel erläutert die Erfindung ohne diese zu begrenzen.

### Ischemie-Reperfusions Modell

Bei jedem chirurgischen Eingriff ergeben sich reversible Ischemien von Organen und Geweben, die durch temporäres Abklemmen von Blutgefässen verursacht werden. Eine solche Ischemie-Situation kann ein einem "low-flow, reflow" Leberperfusionsmodell an der Ratte simuliert werden

In diesem Rattenmodell wird die Leber bei niedriger Flussrate perfundiert, um in der perizentralen Leberregion durch verminderte Sauerstoffzufuhr einen lokalen Sauerstoffmangel, i.e. Anoxie zur erzeugen, Wird die Leber anschließend bei normalen Flussraten reperfundiert, wird wiederum Sauerstoff in die vorher anoxischen Leberregionen eingebracht, was dann zu einer Sauerstoff-abhängigen Radikalbildung und einem entsprechenden Reperfusions-Schaden führt. Durch den dabei verursachten Zelltod vieler Zellen in der perizentralen Leberregion werden zellgebundene Enzyme, z.B. Laktate-hydrogenase und Transaminasen, freigesetzt und können im Perfusat nachgewiesen und quantifiziert werden.

200-250 g schwere Sprague-Dawley Ratten werden über Nacht gefastet. Ein erstes Teil der Tiere erhält eine Kombination von Grüntee-Extrakt und L-Arginin in den Magen appliziert (Gavage). Einer zweiten Gruppe (Kontrolle) wird ein identisches Volumen an Wasser appliziert. Anschließend werden die Ratten mit Phenobarbital betäubt und das Abdomen eröffnet. Eine Teil-Ischemie der Leber wird durchgeführt, wobei Arterie und Portalvene für die drei oberen Leberlappen (ca. 70% der gesamten Lebermasse) für eine Stunde abgeklemmt wird. Anschließend wird die Leber reperfundiert und die operative Wunde geschlossen.

Ein Anstieg der Serum Transaminasen wird nach 1,5; 3; 7 und 24 Stunden gemessen.

In der Kontrollgruppe steigen die Transaminasen nach 7 Stunden um mehr als das 50-fache an, während ein signifikant geringerer Anstieg dieser Enzymaktivitäten unter den Bedingungen der Applikation der Kombination von Grüntee-Extrakt und L-Arginin beobachtet wird.

Dieser Effekt demonstriert die Wirkung der Kombination von Grüntee-Extrakt und L-Arginin in der Prävention von Ischemie-Reperfusions Schäden.

## Patentansprüche

1. Verwendung einer Zusammensetzung enthaltend
a) Grüntee-Extrakt und
b) mindestens eines NO-Donors, der ein Substrat der NO-Synthetase ist, und/oder eines Vorläufers dieses NO-Donors,
zur Herstellung einer Formulierung zur gastro-intestinalen Verabreichung vor chirurgischen Eingriffen, um das Risiko für postoperative Komplikationen zu verringern oder ein solches zu vermeiden.

2. Verwendung einer Zusammensetzung nach Anspruch 1, **dadurch gekennzeichnet, dass** der NO-Donor, der ein Substrat der NO-Synthetase ist, oder der Vorläufer dieses NO-Donors ausgewählt wird aus der Gruppe bestehend aus Arginin, Glutamin, Vorläufern dieser Aminosäuren, Trinitroglycerin, Isosorbitdinitrat, Nitroprussit, Aminoguanidin, Spermin-NO, Spermidin-NO und SIN 1 (3-Morpholinosydnonimin), der physiologisch verträglichen Salze oder deren Kombinationen.

3. Verwendung einer Zusammensetzung nach Anspruch 1, **dadurch gekennzeichnet, dass** es sich bei dem chirurgischen Eingriff um einen elektiven chirurgischen Eingriff oder um einen chirurgischen Notfalleingriff handelt.

4. Verwendung einer Zusammensetzung nach Anspruch 3, **dadurch gekennzeichnet, dass** es sich bei dem elektiven chirurgischem Eingriff um einen gastrointestinalen Eingriff, um Herzchirurgie, um Hals- und Nasenchirurgie, um einen Abdominaleingriff, um Gefäß und/oder Gelenkchirurgie oder um Transplantationen handelt.

5. Verwendung einer Zusammensetzung nach Anspruch 3, **dadurch gekennzeichnet, dass** es sich bei dem chirurgischem Notfalleingriff um Traumachirurgie oder um Eingriffe zur Sanierung eines septischen Fokus handelt.

6. Verwendung einer Zusammensetzung nach Anspruch 1, **dadurch gekennzeichnet, dass** der Grüntee-Extrakt Theanin und Polyphenole aufweist, die sich von Catechinderivaten ableiten.

7. Verwendung einer Zusammensetzung nach Anspruch 6, **dadurch gekennzeichnet, dass** die Catechinderivate ausgewählt werden aus der Gruppe bestehend aus (-)-Epigallocatechin-gallat (EGCg), (-)-Epigallicatechin (EGG), (-)-Epicatechin-gallat (ECg), (+)-Gallocatechin (GC), (-)-Epicatechin (EC), (+)-Catechin (C) und Kombinationen von zwei oder mehreren Bestandteilen davon.

8. Verwendung einer Zusammensetzung nach Anspruch 1, **dadurch gekennzeichnet, dass** der eingesetzte Grüntee-Extrakt durch Behandeln von Blättern des Grüntees mit heißem Wasser erhalten worden ist.

9. Verwendung einer Zusammensetzung nach Anspruch 1, **dadurch gekennzeichnet, dass** Komponente b) Arginin oder ein Arginin-Vorläufer in Form eines Di- oder Tripeptids ist.

10. Verwendung einer Zusammensetzung nach Anspruch 1, **dadurch gekennzeichnet, dass** diese als Komponente c) zusätzlich noch Glycin, einen Glycin-Vorläufer in Form eines Di- oder Tripeptids, der physiologisch verträglichen Salze davon oder deren Kombinationen aufweist.

11. Verwendung einer Zusammensetzung nach Anspruch 1, **dadurch gekennzeichnet, dass** diese zur Verabreichung in weniger als vierundzwanzig Stunden, bevorzugt weniger als zwölf Stunden, insbesondere weniger als sechs Stunden, besonders bevorzugt weniger als drei Stunden vor der Operation vorgesehen ist.

## Claims

1. Use of a composition comprising
a) green tea extract and
b) of at least one NO donor which is a substrate of NO synthetase, and/or of one precursor of this NO donor,
for the preparation of a formulation for gastrointestinal administration before surgical procedures, in order to reduce the risk of postoperative complications or to avert such a risk.

2. Use of a composition according to Claim 1, **characterized in that** the NO donor which is a substrate of NO synthase, or the precursor of this NO donor, is selected from the group consisting of arginine, glutamine, precursors of these amino acids, trinitroglycerin, isosorbite dinitrate, nitroprussite, aminoguanidine, spermine-NO, spermidine-NO and SIN 1 (3-morpholinosydnone imine), of the physiologically tolerated salts or combinations thereof.

3. Use of a composition according to Claim 1, **characterized in that** the surgical procedure is an elective surgical procedure or an emergency surgical procedure.

4. Use of a composition according to Claim 3, **characterized in that** the elective surgical procedure is a gastrointestinal procedure, heart surgery, nose and throat surgery, an abdominal procedure, vascular and/or joint surgery or transplantations.

5. Use of a composition according to Claim 3, **characterized in that** the emergency surgical procedure is trauma surgery or procedures for clearing up a septic focus.

6. Use of a composition according to Claim 1, **characterized in that** the green tea extract includes theanine and polyphenols derived from catechin derivatives.

7. Use of a composition according to Claim 6, **characterized in that** the catechin derivatives are selected from the group consisting of (-)-epigallocatechin gallate (EGCg), (-)-epigallicatechin (EGG), (-)-epicatechin gallate (ECg), (+)-gallocatechin (GC), (-)-epicatechin (EC), (+)-catechin (C) and combinations of two or more constituents thereof.

8. Use of a composition according to Claim 1, **characterized in that** the green tea extract used has been obtained by treating green tea leaves with hot water.

9. Use of a composition according to Claim 1, **characterized in that** component b) is arginine or an arginine precursor in the form of a di- or tripeptide.

10. Use of a composition according to Claim 1, **characterized in that** the latter additionally includes as component c) glycine, a glycine precursor in the form of a di- or tripeptide, of the physiologically tolerated salts thereof or combinations thereof.

11. Use of a composition according to Claim 1, **characterized in that** it is provided for administration in less than twenty-four hours, preferably less than twelve hours, in particular less than six hours, particularly preferably less than three hours, before the operation.

## Revendications

1. Utilisation d'une composition contenant
a) de l'extrait de thé vert et
b) au moins un donneur de NO, qui est un substrat de la NO-synthétase, et/ou un précurseur de ce donneur de NO
pour la production d'une formulation pour administration gastro-intestinale avant interventions chirurgicales, afin de réduire le risque de complications postopératoires ou de l'éviter.

2. Utilisation d'une composition selon la revendication 1, **caractérisée en ce que** le donneur de NO, qui est un substrat de la NO-synthétase ou le précurseur de ce donneur de NO est choisi dans le groupe constitué de l'arginine, de la glutamine, des précurseurs de ces acides aminés, de la trinitroglycérine, du dinitrate d'isosorbitol, de la nitroprussite, de l'aminoguanidine, de la spermine-NO, de la spermidine-NO et de SIN 1 (3-morpholinosydnonimine), de leurs sels physiologiquement compatibles ou de leurs combinaisons.

3. Utilisation d'une composition selon la revendication 1, **caractérisée en ce que** l'intervention chirurgicale est une intervention chirurgicale élective ou une intervention chirurgicale d'urgence.

4. Utilisation d'une composition selon la revendication 3, **caractérisée en ce que** l'intervention chirurgicale élective est une intervention gastro-intestinale, une chirurgie cardiaque, une chirurgie ORL, une intervention abdominale, une chirurgie d'un vaisseau et/ou d'une articulation ou des transplantations.

5. Utilisation d'une composition selon la revendication 3, **caractérisée en ce que** l'intervention chirurgicale d'urgence est une chirurgie traumatologique ou des interventions pour le traitement d'un foyer septique.

6. Utilisation d'une composition selon la revendication 1, **caractérisée en ce que** l'extrait de thé vert présente de la théanine et des polyphénols qui proviennent des dérivés de la catéchine.

7. Utilisation d'une composition selon la revendication 6, **caractérisée en ce que** les dérivés de la catéchine sont choisis dans le groupe composé de (-)-épigallocatéchine gallate (EGCg), de (-)-épigallocatéchine (EGG), de (-)-épicatéchine gallate (ECg), de (+)-gallocatéchine (GC), de (-)-épicatéchine (EC), de (+)-catéchine (C) et de combinaisons de deux de leurs composants ou plus.

8. Utilisation d'une composition selon la revendication 1, **caractérisée en ce que** l'extrait utilisé de thé vert a été obtenu par traitement de feuilles du thé vert avec de l'eau chaude.

9. Utilisation d'une composition selon la revendication 1, **caractérisée en ce que** le composant b) est de l'arginine ou un précurseur de l'arginine sous la forme d'un di ou tripeptide.

10. Utilisation d'une composition selon la revendication 1, **caractérisée en ce que** celle-ci présente comme composant c) également de la glycine, un précurseur de la glycine sous la forme d'un di ou tripeptide, de leurs sels physiologiquement compatibles ou de leurs combinaisons.

11. Utilisation d'une composition selon la revendication 1, **caractérisée en ce que** celle-ci est prévue pour l'administration en moins de vingt-quatre heures, de préférence moins de douze heures, en particulier moins de six heures, plus préférablement moins de trois heures avant l'opération.
